# EUROPÄISCHE PATENTANMELDUNG

(11) **EP 1 197 482 A1**
(43) Veröffentlichungstag der Anmeldung: **17.04.2002**
(21) Anmeldenummer: 00122489.8
(22) Anmeldetag: 14.10.2000
(51) Int. Cl.: C07C 41/26, C07C 43/13

(54) **Verfahren zur Herstellung von 3-Alkoxypropanol**

(71) Anmelder: Degussa AG, 40474 Düsseldorf (DE)
(72) Erfinder: Hahm, Torsten, Dr., 63584 Gründau/Rothenbergen (DE); Ronge, Christian, 63826 Geiselbach (DE); Kuhnen, Manuela, 63454 Hanau (DE); Vanheertum, Rudolf, Dr., 63796 Kahl (DE); Wöll, Wolfgang, 63477 Maintal-Dörnigheim (DE)

(57) **Zusammenfassung**

Verfahren zur Herstellung von 3-Alkoxypropanol durch heterogen katalysiertes Hydrieren von 3-Alkoxypropionaldehyd in alkoholischer Lösung, wobei als Katalysator mindestens ein Metall aus der Gruppe Eisen, Kobalt, Nickel, Kupfer, Silber, Molybdän, Wolfram, Vanadin, Chrom, Rhodium, Palladium, Osmium, Iridium, Ruthenium und/oder Platin auf einem oxidischen Träger eingesetzt wird.

## Beschreibung

Die Erfindung betrifft ein Verfahren zur Herstellung von 3-Alkoxypropanol durch Hydrieren von 3-Alkoxypropionaldehyd.

Zur Herstellung von 3-Alkoxypropanol sind verschiedene Verfahren, die entweder von einem C₂ und C₁-Baustein oder von einem C₃-Baustein, wie z. B. Acrolein, ausgehen, bekannt.

So ist es bekannt, Acrolein in Gegenwart eines Katalysators zunächst zu alkoxylieren, wobei 3-Alkoxypropionaldehyd gebildet wird. Das bei der Alkoxylierung gebildete Reaktionsgemisch enthält nach Abtrennung von nicht umgesetztem Acrolein und ohne Berücksichtigung des Lösungsmittels neben ca. 95 % % 3-Alkoxypropionaldehyd weitere organische Komponenten in geringeren Gewichtsanteilen. Zur Herstellung von 3-Alkoxypropanol wird dieses Reaktionsgemisch in Gegenwart von Hydrierkatalysatoren hydriert.

Der Katalysator kann in suspendierter Form per se, trägergebunden vorliegen oder Bestandteil von Festbettkatalysatoren sein. Auch homogene Katalysatoren können herangezogen werden. Als Suspensionskatalysatoren sind Raney-Nickel, das mit verschiedenen anderen katalytisch wirksamen Metallen dotiert sein kann, sowie Platin auf Aktivkohle bekannt (DE 39 26 136).

Das bekannte Verfahren zur katalytischen Hydrierung weist den Nachteil auf, daß das katalytisch aktive Element in geringen Mengen in Form löslicher Verbindungen in den Produktstrom ausgetragen wird, und somit zusätzliche Arbeitsschritte zur Abtrennung der dadurch verursachten Verunreinigungen notwendig werden. Das ist insbesondere bei Suspensionskatalysatoren, wie zum Beispiel Raney-Nickel, zu beobachten.

Bei Nickel-Festbettkontakten besteht zusätzlich der Nachteil, daß das Produkt durch Nickelverbindungen verunreinigt wird.

Hydrierverfahren können durch die mit ihnen erzielbaren Umsätze, Selektivitäten und Raum-Zeit-Ausbeute charakterisiert werden.

Der Umsatz gibt an, wieviel Mol des Eduktes (hier 3-Alkoxypropionaldehyd) durch die Hydrierung in andere Stoffe umgesetzt werden. Die Angabe erfolgt gewöhnlich in Prozent der eingesetzten Mol des Eduktes.

Die Selektivität des Hydrierverfahrens ist dagegen ein Maß dafür, wieviel Mol des Eduktes in das gewünschte Produkt überführt werden. Für kontinuierliche Hydrierverfahren ist die Raum-Zeit-Ausbeute eine weitere wichtige Kenngröße, die die erzielbare Menge des Produktes pro Zeiteinheit und Reaktionsvolumen angibt.

Bei der großtechnischen Hydrierung von 3-Alkoxypropionaldehyd zu 3-Alkoxypropanol ist für die Wirtschaftlichkeit des Hydrierverfahrens und für die Qualität des Produktes entscheidend, daß Umsatz und Selektivität möglichst nahe bei 100 % liegen. Zwar wird nach der Hydrierung das 3-Alkoxypropanol von dem im Produktstrom enthaltenem Alkohol und restlichen Alkoxypropionaldehyd sowie Nebenprodukten durch Destillation getrennt, jedoch wird diese destillative Trennung durch Rest-Alkoxypropionaldehyd und Nebenprodukte wesentlich erschwert. Je geringer Umsatz und Selektivität sind, umso schlechter ist die erreichbare Produktqualität.

Um 3-Alkoxypropanol wirtschaftlich herstellen zu können, ist es weiterhin wichtig, daß der Katalysator für die Hydrierung von 3-Alkoxypropanal eine hohe Aktivität zeigt.

Eine Aufgabe der Erfindung ist es, ein Verfahren zu finden, bei dem für die Herstellung von 3-Alkoxypropanol eine möglichst geringe Menge an Katalysator notwendig ist.
Es soll mit einem geringen Katalysatorvolumen ein möglichst hoher Umsatz von 3-Alkoxypropanal zu 3-Alkoxypropanol erzielt werden.

Bei Hydrierverfahren werden Umsatz, Selektivität und Raum-Zeit-Ausbeute durch die Eigenschaften des Katalysators und durch die Hydrierbedingungen, wie Reaktionstemperatur, Wasserstoffdruck und Hydrierdauer, oder im Falle kontinuierlicher Hydrierungen durch die Raumgeschwindigkeit LHSV (Liquid Hourly Space Velocity) beeinflußt.

Aus dem Prospekt Engelhard "Exceptional Technologies" 1991 ist es bekannt, aliphatische Carbonylverbindungen in Gegenwart von Ruthenium auf Aluminiumoxid zu den entsprechenden Alkoholen zu hydrieren (Escalit).

Aus dem Prospekt der Degussa "Powder Precious Metal Catalyts" (publiziert 6/95) ist es bekannt, aliphatische Aldehyde zu Alkoholen in Gegenwart Ruthenium-Trägerkatalysatoren zu hydrieren. Als Träger wird dabei Aluminiumoxid angegeben.

Ein wesentliches Qualitätskriterium für die beim Hydrierverfahren eingesetzten Katalysatoren ist ihre Standzeit im Betrieb. Gute Katalysatoren sollten im Laufe der Betriebszeit gleichbleibenden Umsatz und Selektivität bei der Hydrierung von 3-Alkoxypropionaldehyd zu 3-Alkoxypropanol gewährleisten. Hier zeigen bekannte Hydrierverfahren gemäß dem Stand der Technik insbesondere auf der Basis von Nickelkatalysatoren ungenügende Langzeit-Stabilitäten. Dies erfordert einen häufigeren Wechsel der gesamten Katalysatorpackung mit den bekannten Problemen bei der Entsorgung und Aufarbeitung von nickelhaltigen Verbindungen.

Es ist daher eine weitere Aufgabe der vorliegenden Erfindung, ein Hydrierverfahren, das die Nachteile der Verfahren gemäß dem Stand der Technik nicht aufweist, zu entwickeln.

Gegenstand der Erfindung ist ein Verfahren zur Herstellung von 3-Alkoxypropanol, welches dadurch gekennzeichnet ist, daß man bei dem heterogen katalysierten Hydrieren von 3-Alkoxypropionaldehyd in alkoholischer Lösung bei einer Temperatur von 30 bis 120 °C, einem Wasserstoffdruck von 5 bis 300 bar und einem pH-Wert von 2,5 bis 7,0 als Katalysator einen Trägerkatalysator verwendet, der aus einer oxidischen Phase, vorzugsweise einer oxidischen Phase, die in saurem Milieu beständig ist, besteht und auf dem mindestens ein Metall aus der Gruppe Eisen, Kobalt, Nickel, Kupfer, Silber, Molybdän, Wolfram, Vanadin, Chrom, Rhodium, Palladium, Osmium, Iridium Ruthenium und/oder Platin, insbesondere Ruthenium, Platin und/oder Nickel, in einer Menge von 0,1 bis 5 Gew.-%, bezogen auf die oxidische Phase, in feinverteilter Form vorliegt.

In einer bevorzugten Ausführungsform der Erfindung kann die Temperatur 40 bis 100 °C, insbesondere 50 bis 80 °C betragen. Der Druck kann bevorzugt 10 bis 150, insbesondere 40 bis 100 bar betragen. Der pH-Wert kann bevorzugt 3 bis 6, insbesondere 4 bis 5 betragen.

Als oxidische Phase kann man einen Stoff aus der Gruppe Titandioxid, SiO₂, Al₂O₃ und/oder deren Mischoxide sowie Aluminiumsilikate, MgO, Zeolithe oder Zirkondioxid verwenden.

Derartige Stoffe sind beispielsweise beschrieben in Catalyst Supports and Supported Catalysts von Alvin B, Stiles Verlag, Butterworths 1987, Kapitel 2 und 3.

Vorzugsweise wird eine oxidische Phase, die in saurem Milieu beständig ist, verwendet. Derartige oxidische Phasen können Stoffe aus der Gruppe Titandioxid, SiO₂ und/oder deren Mischoxide sowie Aluminiumsilikate, MgO, Zeolithe oder Zirkondioxid sein. Aluminiumoxid weist eine geringere Säurebeständigkeit auf.

In einer bevorzugten Ausführungsform der Erfindung können als oxidische Phase Oxide sowie Mischoxide aus Aluminium sowie insbesondere Titan und/oder Silizium eingesetzt werden.

Als Titandioxid kann ein pyrogen hergestelltes, insbesondere durch Flammenhydrolyse hergestelltes Titandioxid eingesetzt werden.

Als Titanoxid kann man beispielsweise ein durch Flammenhydrolyse aus Titantetrachlorid gewonnenes sogenanntes pyrogenes Titandioxid mit einer BET-Oberfläche von 40 bis 60 m²/g und einem Gesamtporenvolumen von 0,25 bis 0,75 ml/g verwenden, das eine mittlere Größe der Primärteilchen von 20 nm, eine Dichte von 3,7 g/cm³ und eine Röntgenstruktur aus 20 bis 40 % Rutil und 80 bis 60 % Anatas aufweist und dessen Verunreinigungen an Siliciumdioxid, Aluminiumoxid und Eisenoxid unter 0,5 Gew.-% liegen. Pyrogenes Titanoxid wie das Titandioxid P 25 von Degussa-Hüls ist als Träger für die katalytisch aktive Komponente besonders geeignet. Es weist eine hohe spezifische Oberfläche nach BET von im Mittel 50 m²/g (gemessen nach DIN 66131) auf.

Die Oxide können zu Formkörpern wie zum Beispiel Pellets, Granulat oder Strangpreßlingen verarbeitet werden.

Die Beschichtung der oxidischen Phase kann mittels der Incipient Wetness Method, publiziert in "Preparation of Catalyst", Delmon, B., Jacobs, P.A., Poncald, G. (eds.), Amsterdam Elsevier, 1976, Seite 13, erfolgen.

Hierzu wird die Wasseraufnahmekapazität des Trägers bestimmt. Danach wird eine wäßrige Rutheniumchloridlösung mit einer Konzentration entsprechend der späteren Rutheniumbeschichtung hergestellt. Der Träger wird entsprechend der Wasseraufnahmekapazität mit wäßrigen Rutheniumchlorid beladen. Anschließend wird der beladene Träger, vorzugsweise bei 20 bis 100 °C bei Normaldruck in Inertgasatmosphäre, wie Stickstoff, Neon, Helium, Argon oder Luft, getrocknet, mit Wasserstoff, vorzugsweise bei einer Temperatur von 100 bis 500 °C in einer Zeit von 20 Min. bis 24 Std. mit einer Wasserstoff-Konzentration von 1 bis 100 % im Gemisch mit Stickstoff reduziert und gegebenfalls chloridfrei, vorzugsweise < 100 ppm Cl⁻, gewaschen.

Die Alkoxy-Gruppe des Alkoxy-propanals kann Methoxy-, Ethoxy-, Propoxy-, Iso-propoxy-, Butoxy-, Isobutoxy-, Pentoxy-, Isopentoxy u.a. sein.

In einer weiteren Ausführungsform der Erfindung kann man eine Nachhydrierung durchführen. Diese Nachhydrierung kann bei einer Temperatur von 100 bis 180 °C, einem Druck von 5 bis 300 bar und einem pH-Wert von 2,5 bis 7,0 durchgeführt werden.

Die Nachhydrierung kann mit denselben Katalysatoren durchgeführt werden. Auch die Verwendung von Aktivkohle als Katalysatorträger ist dabei möglich.

Die Nachhydrierung hat den Vorteil, daß die Menge an weiteren Carbonylverbindungen, die destillativ nur schwer abtrennbar sind, vermindert wird. Weiterhin werden ebenfalls gebildete Acetate, wie Acroleindialkylacetat oder Trialkoxypropan vermindert.

Die Nachhydrierung kann vorteilhaft nach einem Umsatz von 50 bis 95 % des 3-Alkoxypropanals durchgeführt werden.

Das erfindungsgemäße Verfahren weist den Vorteil auf, daß ein höherer Umsatz auf Grund der höheren Aktivität des Katalysators erzielt werden kann.

Weiterhin weisen die eingesetzten Katalysatoren eine längere Standzeit auf.

### Beispiele

Die Katalysatoren werden unter stationären Bedingungen getestet, um auch Aussagen über das Langzeitverhalten machen zu können. Die Hydrierung wird kontinuierlich in einer Rieselbettanlage mit 140 ml Reaktorvolumen durchgeführt. Die Anlage besteht aus einer Flüssigkeitsvorlage, dem Flüssigkeitsreaktor und einem Flüssigkeitsabscheider. Die Reaktionstemperatur wird über einen Wärmeträger-Öl-Kreislauf eingestellt. Druck und Wasserstoffstrom werden elektronisch geregelt. Die wäßrige Alkoxypropionaldehyd-Lösung wird dem Wasserstoffstrom mit einer Pumpe zudosiert und das Gemisch am Kopf des Reaktors aufgegeben (Rieselbett-Fahrweise). Nach Durchlaufen des Reaktors wird das gebildete Produkt in regelmäßigen Abständen aus dem Abschneider entnommen. Die Konzentration des Alkoxy-propionaldehyd in der Eduktlösung beträgt in allen Fällen 10 Gew.-%, die Temperatur 40°C, der Druck 40 bar, die Flüssigkeitsbelastung LHSV ist 1 h⁻¹.

Herstellvorschrift der Katalysatoren:
1. Es wird die Wasseraufnahme des Trägers in g H₂O pro 100 g Träger bestimmt.
2. Für die Beladung von 250 ml Träger werden 11.8 g RuCl₃ in 145 g destilliertem Wasser gelöst.
3. 250 ml Träger werden im Dragierkessel vorgelegt und im rotierenden Kessel mit der RuCl₃-Lösung übergossen.
4. Der beschichtete Träger wird 16 h an der Luft getrocknet und anschließend im Rohrofen auf 200°C aufgeheizt.
5. Der Katalysator wird bei 200°C über 8 h mit Wasserstoff reduziert.
6. Der reduzierte Katalysator wird dreimal mit jeweils 40 ml destilliertem Wasser chloridfrei gewaschen.

Die eingesetzten Träger sind wie folgt charakterisiert:
Träger 1: Silikagel von Grace (0,8 -1,2 mm)
   Bezeichnung: V432
Träger 2: Aktivkohle von Norit (Durchmesser 2,3 mm)
   Bezeichnung: Norit CNR 115 (Olivenkerne)
Träger 3: Aktivkohle von Norit (Durchmesser 0,8 mm)
   Bezeichnung: Norit ROX (Torfkohle)
Träger 4: Titandioxid P25 (pyrogen mittels Flammenhydrolyse hergestellt von Degussa-Hüls AG)
Träger 5: Al₂O₃ von Rhône-Poulenc (Durchmesser 1,1 -1,3 mm)
   Bezeichnung: Spheralite 521

Bei der Beschichtung der Träger werden die folgenden Bedingungen eingehalten:

| | Träger | Wasser-Aufnahme [g/100g Träger] | Träger [g] | RuCl₃ [g] | Wasser [g] |
|---|---|---|---|---|---|
| Träger 1 | SiO₂ V432 | 126 | 115 | 11,8 | 145 |
| Träger 2 | Norit 1 Extra | 67 | 105 | 10,8 | 56 |
| Träger 3 | Norit ROX 0,8 | 80 | 107 | 11,1 | 68 |
| Träger 4 | TiO₂ EP0 535 565 | 25 | 100 | 10,2 | 14 |
| Träger 5 | Al₂O₃ | 67 | 101 | 10,3 | 57 |

Im Gegensatz zu bekannten Katalysatoren zeigen die erfindungsgemäßen Rutheniumkatalysatoren auf oxidischen Trägern keinerlei Desaktivierung. Der Umsatz bleibt konstant. Insbesondere die Katalysatoren mit Ruthenium auf SiO₂ und TiO₂ zeigen eine sehr hohe Aktivität.

## Patentansprüche

1. Verfahren zur Herstellung von 3-Alkoxypropanol, **dadurch gekennzeichnet, daß** man bei dem heterogen katalysierten Hydrieren von 3-Alkoxypropionaldehyd in alkoholischer Lösung bei einer Temperatur von 30 bis 120 °C, einem Wasserstoffdruck von 5 bis 300 bar, und einem pH-Wert von 2,5 bis 7,0 als Katalysator einen Trägerkatalysator verwendet, der aus einer oxidischen Phase besteht und auf dem mindestens ein Metall aus der Gruppe Eisen, Kobalt, Nickel, Kupfer, Silber, Molybdän, Wolfram, Vanadin, Chrom, Rhodium, Palladium, Osmium, Iridium, Ruthenium und/oder Platin in einer Menge von 0,1 bis 5 Gew.-%, bezogen auf die oxidische Phase, in feinverteilter Form vorliegt.

2. Verfahren nach Anspruch 1, **dadurch gekennzeichnet, daß** man als oxidische Phase einen Stoff aus der Gruppe Titandioxid, SiO₂, Al₂O₃ und/oder deren Mischoxide sowie Aluminiumsilikate, MgO, Zeolithe oder Zirkonoxid verwendet.

3. Verfahren nach Anspruch 1, **dadurch gekennzeichnet, daß** man eine Nachhydrierung durchführt.
